# EUROPÄISCHE PATENTANMELDUNG

(11) **EP 0 582 923 A1**
(43) Veröffentlichungstag der Anmeldung: **16.02.1994**
(21) Anmeldenummer: 93112313.7
(22) Anmeldetag: 31.07.1993
(51) Int. Cl.: C07C 41/46, C07C 43/11, C08K 5/13

(54) **Thermoxidative Stabilisierung von Oxalkylenglykolalkylethern mit sterisch gehinderten Phenolen**

(30) Priorität: 08.08.1992 DE 4226288
(71) Anmelder: HOECHST AKTIENGESELLSCHAFT, D-65926 Frankfurt am Main (DE)
(72) Erfinder: Geymayer, Peter, Dr., D-84508 Burgkirchen (DE); Glass, Brunhilde, D-84489 Burghausen (DE); Streitberger, Horst, D-84503 Altötting (DE)

(57) **Zusammenfassung**

Nach dem beschriebenen Verfahren werden den Oxalkylenglykolalkylethern spezielle phenolische Verbindungen einverleibt, die eine hohe und lang anhaltende thermooxidative Stabilität der Ether bewirken. Die beschriebenen Mischungen bestehen im wesentlichen aus mindestens einem Oxalkylenglykolalkylether als Hauptkomponente und mindestens einer der angegebenen phenolischen Verbindungen als thermooxidativer Stabilisator.

## Beschreibung

Die Erfindung betrifft ein verfahren zur thermooxidativen Stabilisierung von Oxalkylenglykolalkylethern, insbesondere Oxalkylenglykoldialkylethern. Die Erfindung betrifft ferner eine Mischung bestehend aus mindestens einem Oxalkylenglykolalkylether, insbesondere Oxalkylenglykoldialkylether, und einem Stabilisierungsmittel gegen den thermooxidativen Abbau dieser Ether.

Oxalkylenglykolalkylether stellen bekanntlich vorteilhafte Lösungs-, Reinigungs- und Verdünnungsmittel dar. In dieser Funktion werden sie beispielsweise zur Herstellung von Lacken, Holzbeizen, Klebstoffen und hydraulischen Flüssigkeiten eingesetzt sowie zur Entfernung von sauren Gasen (das sind insbesondere Schwefeldioxid, Schwefelwasserstoff und Kohlendioxid) und Lösemitteln aus entsprechenden Gasströmen (Abgasen).

Es ist ferner bekannt, daß Oxalkylenglykolalkylether in Gegenwart von oxidierenden Komponenten wie Sauerstoff oder Luft Peroxide bilden, aus denen durch weiteren Abbau verfärbend und/oder korrosiv wirkende Zersetzungsprodukte entstehen wie Aldehyde, Ester und Säuren, und daß die Bildung von Peroxiden und deren Abbau durch Wärme gefördert und beschleunigt wird.

Gleichzeitige Anwesenheit von Luft und Wärme tritt zum Beispiel bei der Entfernung von Schwefeldioxid aus Gasströmen auf, die bei der Verbrennung fossiler Brennstoffe wie Öl, Gas und Kohle und bei der Erzgewinnung und Celluloseherstellung entstehen. Die Reinigung dieser Gase (Rauchgase) erfolgt kurz zusammengefaßt in der Weise, daß man das zu reinigende Gas im sogenannten Rauchgaswäscher der Rauchgasentschwefelungsanlage, in der eine Temperatur von etwa 20 bis 50 °C vorliegt, mit vorzugsweise Oxalkylenglykoldialkylethern in Kontakt bringt, wobei das Schwefeldioxid absorbiert wird. Zur Desorption wird der mit dem sauren Gas beladene Oxalkylenglykoldialkylether in der Regel bei einer Temperatur von etwa 90 bis 120 °C gestrippt. Bei beiden Prozessen ist die Anwesenheit von Luft in der Regel nicht zu vermeiden. Hier wirken also hohe Temperatur und Luft (als oxidierendes Medium) gleichzeitig auf die eingesetzten Oxalkylenglykoldialkylether ein. Dazu kommt noch, daß katalytisch wirkende Substanzen wie SO₃ und/oder H₂SO₄ anwesend sind, was den erwähnten Abbau der Ether noch weiter fördert. Die genannte Rauchgasreinigung stellt also besonders hohe Ansprüche an die Stabilisierung von Oxalkylenglykolalkylethern.

Es sind bereits zahlreiche als Antioxidantien bezeichnete organische Verbindungen bekannt, insbesondere solche phenolischer Art, deren Einsatz überall dort empfohlen wird, wo mit Peroxidbildung oder anderen Oxidationsreaktionen zu rechnen ist. Diese Verbindungen werden auch zur Stabilisierung von Oxalkylenglykolalkylethern eingesetzt. In der europäischen Patentschrift 0 289 935 B1 ist ein Gemisch aus mindestens einer organischen Schwefelverbindung und mindestens einer organischen Stickstoffverbindung als Stabilisator für Oxalkylenglykolalkylether beschrieben. Im Falle der Rauchgasreinigung mit diesen Ethern läßt auch dieser Stabilisator noch zu wünschen übrig.

Die Aufgabe der Erfindung besteht also darin, ein Stabilisierungsmittel für Oxalkylenglykolalkylether vorzuschlagen, insbesondere für Oxalkylenglykoldialkylether, das diesen Ethern vor allem beim Einsatz zur Reinigung von Rauchgas die erforderlich thermooxidative Stabilität verleiht.

Das erfindungsgemäße Verfahren zur thermooxidativen Stabilisierung von Oxalkylenglykolalkylethern ist dadurch gekennzeichnet, daß den Ethern mindestens eine phenolische Verbindung der nachstehenden Formeln (1) und (2) in einer wirksamen Menge einverleibt wird, wobei Formel (1) lautet: 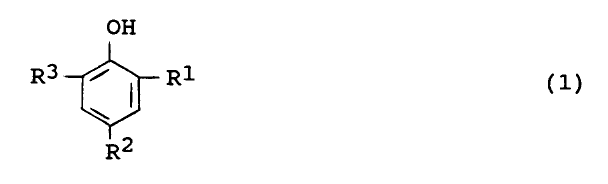
worin bedeuten: R¹ und R² C₁ bis C₆-Alkyl, vorzugsweise C₁ bis C₄-Alkyl, und R³ C₁ bis C₆-Alkyl, vorzugsweise C₁ bis C₄-Alkyl, oder einen Rest der Formel 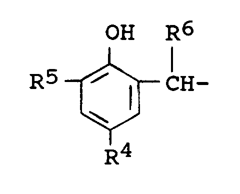
worin R⁴ und R⁵ C₁ bis C₆-Alkyl sind, vorzugsweise C₁ bis C₄-Alkyl, und R⁶ H ist oder C₁ bis C₆-Alkyl, vorzugsweise C₁ bis C₄-Alkyl,
und Formel (2) lautet: 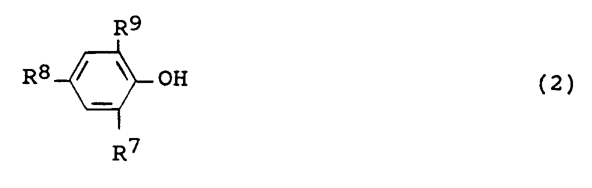
worin bedeuten: R⁷ und R⁹ C₁ bis C₆-Alkyl, vorzugsweise C₁ bis C₄-Alkyl, und R⁸ C₁ bis C₆-Alkyl, vorzugsweise C₁ bis C₄-Alkyl, oder einen Rest der Formel 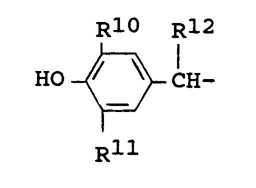
worin R¹⁰ und R¹¹ C₁ bis C₆-Alkyl sind, vorzugsweise C₁ bis C₄-Alkyl, und R¹² H ist oder C₁ bis C₆-Alkyl, vorzugsweise C₁ bis C₄-Alkyl.

Die genannten Alkylreste können gleich oder verschieden sein und verzweigt oder unverzweigt. Sie sind vorzugsweise Methyl oder tert.-Butyl. Von den erfindungsgemäß vorgeschlagenen speziellen phenolischen Verbindungen sind jene der Formel (1) bevorzugt.

Die nachstehenden Formeln (3) bis (6) stellen Beispiele für bevorzugte Vertreter der erfindungsgemäß vorgeschlagenen ausgewählten phenolischen Verbindungen dar: 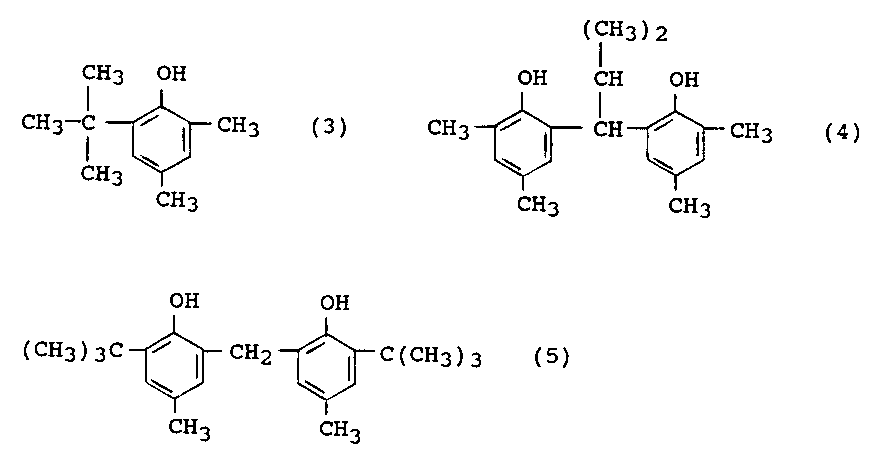 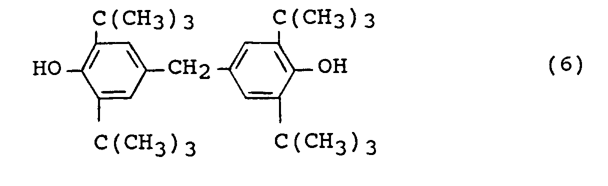
Beim erfindungsgemäßen Verfahren werden also die angegebenen Verbindungen (die im Handel erhältlich sind) den zu stabilisierenden Ethern in einer wirksamen Menge zugesetzt oder eingemischt, gegebenenfalls unter Rühren. Das Zusetzen, Einmischen oder Einverleiben, gegebenenfalls unter Rühren, wird im allgemeinen bei Raumtemperatur (das sind etwa 15 bis etwa 25 °C) durchgeführt. In der Regel lösen sich die Verbindungen im Oxalkylenglykolalkylether relativ schnell auf. Die stabilisierende Wirkung wird durch Zusatz von mindestens einer der Verbindungen erreicht, es können auch zwei oder mehr Verbindungen zugesetzt werden. Die für eine gute Stabilisierung der Ether gegen thermooxidativen Abbau erforderliche Menge an Stabilisierungsmittel (das ist eine Verbindung allein oder eine Mischung von Verbindungen) kann in weiten Grenzen variieren und hängt vor allem von der vorliegenden Temperatur und der Anwesenheit von Luft oder Sauerstoff und/oder von chemisch oder katalytisch aktiven Komponenten wie Schwefelsäure und dergleichen ab. Sie beträgt im allgemeinen 0,05 bis 10 Gew.-%, vorzugsweise 0,1 bis 5 Gew.-%, Gewichtsprozente bezogen auf die (stabilisierte) Mischung.

Die erfindungsgemäße, thermooxidativ stabilisierte Mischung besteht demnach im wesentlichen aus mindestens einem Oxalkylenglykolalkylether als Hauptkomponente oder Hauptmenge und mindestens einer der Verbindungen der Formeln (1) und (2), vorzugsweise der Formeln (3) bis (6), in einer wirksamen Menge als thermooxidativer Stabilisator, wobei die wirksame Menge im allgemeinen 0,05 bis 10 Gew.-% beträgt, vorzugsweise 0,1 bis 5 Gew.-%, Gewichtsprozente bezogen auf die Mischung. Anders ausgedrückt besteht die erfindungsgemäße Mischung im wesentlichen aus 0,05 bis 10 Gew.-%, vorzugsweise 0,1 bis 5 Gew.-%, von mindestens einer Verbindung der Formeln (1) und (2), vorzugsweise der Formeln (3) bis (6), und mindestens einem Oxalkylenglykolalkylether, der im wesentlichen den Rest auf 100 Gew.-% darstellt, Gewichtsprozente bezogen auf das Gewicht der (fertigen, stabilisierten) Mischung. Die erfindungsgemäße Mischung wird vorzugsweise wie oben beschrieben hergestellt, das heißt durch Zusammenmischen der Komponenten gegebenenfalls unter Rühren und im allgemeinen bei Raumtemperatur.

Unter Oxalkylenglykolalkylether werden sowohl die Monoalkylether als auch die Dialkylether verstanden, wobei Alkyl vorzugsweise ein niedriger Alkylrest und die Oxalkylengruppe vorzugsweise Polyoxethylen und/oder Polyoxpropylen ist. Die erfindungsgemäß zu stabilisierenden Oxalkylenglykolalkylether sind vorzugsweise solche der nachstehenden Formel

R¹³O(CH₂CHR¹⁴O)ₙR¹⁵

worin R¹³ für einen Alkylrest mit 1 bis 4 C-Atomen, R¹⁴ für H oder CH₃, n für eine Zahl von 1 bis 20, vorzugsweise 2 bis 10, und R¹⁵ für H oder einen Alkylrest mit 1 bis 4 C-Atomen steht,
wobei die Dialkylether bevorzugt sind. Bezüglich der Oxethylen- und Oxpropylenreste sind die nur Oxethylenreste enthaltenden Ether bevorzugt. Für den erwähnten Einsatz von Oxalkylenglykoldialkylethern zur Entfernung von SO₂ aus Rauchgas wird oft auch eine Mischung von Dialkylethern von vorzugsweise Polyethylenglykolen mit 2 bis 10 Ethylenoxideinheiten eingesetzt.

Die mit den erfindungsgemäß vorgeschlagenen, im Handel erhältlichen phenolischen Verbindungen oder phenolischen Antioxidantien versetzten Oxalkylenglykolalkylether weisen eine unerwartet hohe und lang anhaltende thermooxidative Stabilität auch dann auf, wenn sie den harten Bedingungen der Rauchgasbehandlung zwecks Absorption von sauren Gasen ausgesetzt sind. Die Bedingungen sind deswegen in der Tat als hart zu bezeichnen, weil gleichzeitig relativ hohe Temperaturen, mehr oder weniger Luft oder Sauerstoff als oxidierendes Medium und Schwefeltrioxid und/oder Schwefelsäure vorliegen.

Die Erfindung wird nun an Beispielen und Vergleichsbeispielen noch näher erläutert.

### Beispiel 1

0,25 g von der Verbindung der Formel (3) und - zur Nachahmung der Bedingungen bei der Rauchgaswäsche - 0,05 g von einer 98gew.%igen Schwefelsäure wurden in 50 g einer Ethermischung aus 15 Gew.-% Dimethyltriethylenglykol, 60 Gew.-% Dimethyltetraethylenglykol, 20 Gew.-% Dimethylpentaethylenglykol und 5 Gew.-% Dimethylhexaethylenglykol eingemischt. Die Prüfung der Mischung auf die thermooxidative Stabilität erfolgte nach DIN 51 780 bei einer Temperatur von 120 °C und einem Sauerstoffdruck von 400 kPa (DIN = Deutsche Industrie-Norm). Aus einer Druck-Zeit-Kurve wurde diejenige Zeitdauer in Minuten festgestellt, die vom Anfangszustand 120 °C und 400 kPa bis zum Zustand 120 °C und 200 kPa verging (das ist 50%ige Druckabnahme, die im wesentlichen aus Sauerstoffverbrauch resultiert). Diese Zeit ist das Maß für die thermooxidativ stabilisierende Wirkung der eingesetzten Verbindungen. Eine stabilisierende Verbindung oder ein Gemisch von solchen Verbindungen ist um so wirksamer je länger diese Zeit ist und umgekehrt.

### Beispiel 2

2,5 g von der Verbindung der Formel (4) und 0,05 g von einer 98gew.%igen Schwefelsäure wurden in 50 g der Ethermischung von Beispiel 1 eingemischt. Die Mischung wurde wie in Beispiel 1 getestet.

### Beispiel 3

1,25 g von der Verbindung der Formel (5) und 0,05 g von einer 98gew.%igen Schwefelsäure wurden in 50 g der Ethermischung von Beispiel 1 eingemischt. Die Mischung wurde wie in Beispiel 1 getestet.

### Beispiel 4

0,25 g von der Verbindung der Formel (6) und 0,05 g von einer 98gew.%igen Schwefelsäure wurden in 50 g der Ethermischung von Beispiel 1 eingemischt. Die Mischung wurde wie in Beispiel 1 getestet.

### Vergleichsbeispiel

Es wurden 50 g der Ethermischung von Beispiel 1 als solche, das heißt ohne eine stabilisierende Verbindung und ohne Schwefelsäure, den beschriebenen Bedingungen von 120 °C und 400 kPa Sauerstoffdruck ausgesetzt, und es wurde - wie oben beschrieben - die Zeit nach 50%igem Druckabfall notiert.

In der nachstehenden Tabelle sind die Ergebnisse der Beispiele 1 bis 4 und des Vergleichsbeispiels zusammengefaßt:

| Beispiel/Verbindung | Zeit in Minuten nach 50%igem Druckabfall gemäß DIN 51 780 |
|---|---|
| 1 / (3) | 1 200 |
| 2 / (4) | 3 100 |
| 3 / (5) | 4 100 |
| 4 / (6) | 1 600 |
| Vergleichsbeispiel | 190 |

Wie die Ergebnisse der Beispiele und des Vergleichsbeispiels zeigen, ist die thermooxidative Stabilität eines erfindungsgemäß stabilisierten Oxalkylenglykolalkylethers (Beispiele 1 bis 4) etwa 6 bis 20 mal so hoch wie die von einem nicht stabilisierten (Vergleichsbeispiel).

## Patentansprüche

1. Verfahren zur thermooxidativen Stabilisierung von Oxalkylenglykolalkylethern, dadurch gekennzeichnet, daß den Ethern mindestens eine phenolische Verbindung der nachstehenden Formeln (1) und (2) in einer wirksamen Menge einverleibt wird, wobei Formel (1) lautet: 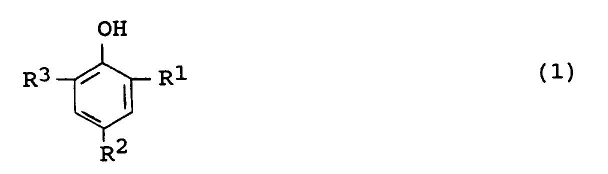 worin bedeuten: R¹ und R² C₁ bis C₆-Alkyl und R³ C₁ bis C₆-Alkyl oder einen Rest der Formel 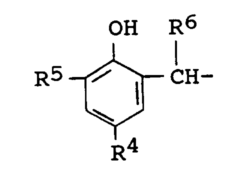 worin R⁴ und R⁵ C₁ bis C₆-Alkyl sind und R⁶ H ist oder C₁ bis C₆-Alkyl,
und Formel (2) lautet: 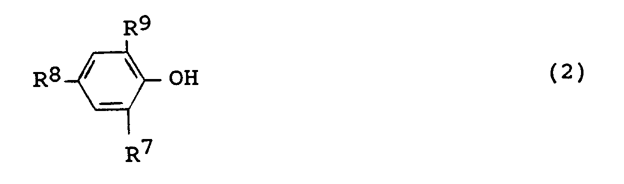 worin bedeuten: R⁷ und R⁹ C₁ bis C₆-Alkyl und R⁸ C₁ bis C₆-Alkyl oder einen Rest der Formel 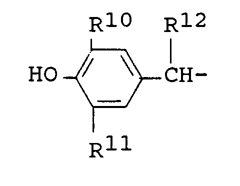 worin R¹⁰ und R¹¹ C₁ bis C₆-Alkyl sind und R¹² H ist oder C₁ bis C₆-Alkyl.

2. Verfahren nach Anspruch 1, wobei in Formel (1) bedeuten: R¹ und R² C₁ bis C₄-Alkyl und R³ C₁ bis C₄-Alkyl oder einen Rest der Formel 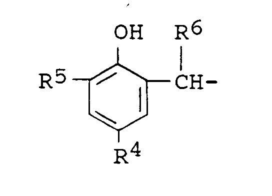 worin R⁴ und R⁵ C₁ bis C₄-Alkyl sind und R⁶ H ist oder C₁ bis C₄-Alkyl,
und in Formel (2) bedeuten: R⁷ und R⁹ C₁ bis C₄-Alkyl und R⁸ C₁ bis C₄-Alkyl oder einen Rest der Formel 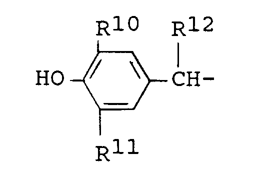 worin R¹⁰ und R¹¹ C₁ bis C₄-Alkyl sind und R¹² H ist oder C₁ bis C₄-Alkyl.

3. Verfahren nach Anspruch 1, wobei den Ethern mindestens eine der nachstehenden phenolischen Verbindungen (3) bis (6) in einer wirksamen Menge einverleibt wird:

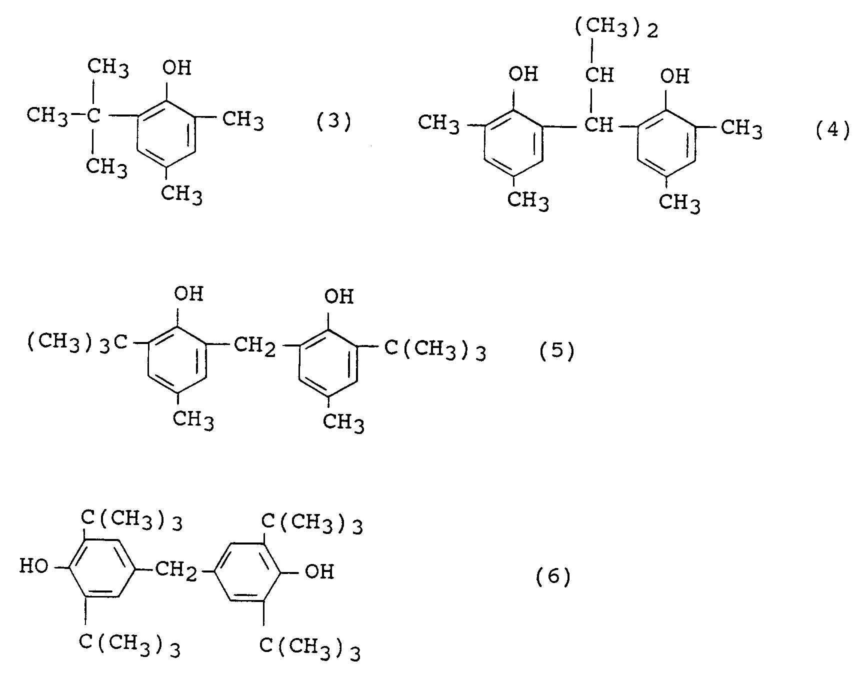

4. Verfahren nach einem oder mehreren der Ansprüche 1 bis 3, wobei den Ethern die phenolische(n) Verbindung(en) in einer Menge von 0,05 bis 10 Gew.-%, vorzugsweise 0,1 bis 5 Gew.-%, einverleibt wird (werden), Gewichtsprozente bezogen auf die stabilisierte Mischung.

5. Verfahren nach einem oder mehreren der Ansprüche 1 bis 4, wobei als Oxalkylenglykolalkylether solche der nachstehenden Formel eingesetzt werden
R¹³O(CH₂CHR¹⁴O)ₙR¹⁵
worin R¹³ für einen Alkylrest mit 1 bis 4 C-Atomen, R¹⁴ für H oder CH₃, n für eine Zahl von 1 bis 20 und R¹⁵ für H oder einen Alkylrest mit 1 bis 4 C-Atomen steht.

6. Verfahren nach einem oder mehreren der Ansprüche 1 bis 4, wobei als Oxalkylenglykolalkylether solche der nachstehenden Formel eingesetzt werden
R¹³O(CH₂CH₂O)ₙR¹⁵
worin R¹³ für einen Alkylrest mit 1 bis 4 C-Atomen, n für eine Zahl von 2 bis 10 und R¹⁵ für einen Alkylrest mit 1 bis 4 C-Atomen steht.

7. Thermooxidativ stabilisierte Mischung bestehend im wesentlichen aus 0,05 bis 10 Gew.-%, vorzugsweise 0,1 bis 5 Gew.-%, von mindestens einer phenolischen Verbindung der im Anspruch 1 angegebenen Formeln (1) und (2) und mindestens einem Oxalkylenglykolalkylether, der im wesentlichen den Rest auf 100 Gew.-% darstellt, Gewichtsprozente bezogen auf das Gewicht der Mischung.

8. Mischung nach Anspruch 7, wobei die phenolische Verbindung den im Anspruch 3 angegebenen Formeln (3) bis (6) entspricht.

9. Mischung nach Anspruch 7 oder 8, wobei der Oxalkylenglykolalkylether ein solcher der nachstehenden Formel ist
R¹³O(CH₂CHR¹⁴O)ₙR¹⁵
worin R¹³ für einen Alkylrest mit 1 bis 4 C-Atomen, R¹⁴ für H oder CH₃, n für eine Zahl von 1 bis 20 und R¹⁵ für H oder einen Alkylrest mit 1 bis 4 C-Atomen steht.

10. Mischung nach Anspruch 7 oder 8, wobei der Oxalkylenglykolalkylether ein solcher der nachstehenden Formel ist
R¹³O(CH₂CH₂O)ₙR¹⁵
worin R¹³ für einen Alkylrest mit 1 bis 4 C-Atomen, n für eine Zahl von 2 bis 10 und R¹⁵ für einen Alkylrest mit 1 bis 4 C-Atomen steht.
